# EUROPEAN PATENT APPLICATION

(11) **EP 2 497 454 A1**
(43) Date of publication of application: **12.09.2012**
(21) Application number: 11001991.6
(22) Date of filing: 10.03.2011
(51) Int. Cl.: A61K 6/083

(54) **Dental composition**

(71) Applicant: Dentsply DeTrey GmbH, 78467 Konstanz (DE)
(72) Inventor: Stelzig, Simon, 78465 Konstanz (DE); Klee, Joachim, 78315 Radolfzell (DE); Facher, Andreas, 8543 Gundetswil (CH); Weber, Christoph, 78464 Konstanz (DE)
(74) Representative: Hartz, Nikolai

(57) **Abstract**

Dental composition comprising
(i) a water-soluble polymerizable compound of the following formula (1),

AXₙ (1)

wherein
A
is a straight chain or branched linker group containing at least n nitrogen atoms, whereby the linker group has polyamide, polyoxyalkylene and/or polyalkylene imine repeating units and optionally one or more acidic groups,
X
are moieties containing a polymerizable double bond, which X may be the same or different and are represented by the following formula (2), whereby a moiety X of formula (2) is linked to the nitrogen atom of an amino group or a carboxamide group of A, 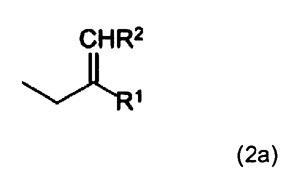 wherein
R¹ and R²
are independent from each other and represent a hydrogen atom, a C₁₋₆ alkyl group or a group -(CH₂)ₘ-COOM, wherein M is a hydrogen atom or a metal atom, and m is an integer of from 0 to 6,
L
is a bond or a C₁₋₆ alkylene group; and
n
is an integer of at least 1;
provided that at least one X cannot be a (meth)acryl group; and
(ii) an initiator system;
(ix) optionally a polyacidic polymer; and
(x) optionally water and/or a water soluble solvent,
(xi) optionally a particulate filler.

## Description

### Field of the invention

The present invention relates to a dental composition containing a specific hydrolysis-stable water-soluble compound. Moreover, the present invention relates to the use of the specific hydrolysis-stable compound for the preparation of a dental composition, in particular a dental adhesive or dental restorative composition. The specific hydrolysis-stable water-soluble compound of the present invention is resistant to an acidic medium so that the dental composition of the present invention may be formulated as an acidic one-component composition, or as a dental cement.

### Background of the Invention

Hard dental tissue having suffered damage due to dental caries is conventionally restored by a procedure in which an indirect restoration such as a crown, a bridge, an inlay, or an onlay, is adhered on the damaged portion of the hard dental tissue with a specific dental composition such as a dental resin cement. Alternatively, damaged hard dental tissue may be restored by using a direct restorative material which is applied as an uncured dental composition such as a dental composite, and hardened.

A dental resin cement and a dental composite are required to have low shrinkage, sufficient adhesion and high material strength. Otherwise, not only the dental composition may be released from the hard dental tissue after some time under the severe conditions of the oral environment, but also a gap may be produced at an interface between the dental composition and the teeth, and bacteria may invade onto the exposed surfaces and impose an adverse effect on dental pulp. Given that the use of a dental primer increases the complexity of a dental procedure, a simple adhesion procedure is desired which uses a dental composition which does not require a primer treatment for such various adherents.

Moreover, since adhesion of a dental composition to hard dental tissue requires the presence of acidic groups in the composition, the dental composition desirably has a high hydrolysis stability in order to avoid degradation of the composition during storage or when applied to hard dental tissue.

Japanese Patent Publication No. 2006-512466A discloses a resin cement which does not require a primer. The polymerizable composite material comprises at least one multifunctional monomer containing an acid in a concentration range of about 10-85% by weight, a non-reactive filler in a concentration range of about 1-80% by weight, a polymerization system in a concentration range of about 1.5-25% by weight, and water in a concentration range of about 0.1-25% by weight. However, since such the composition uses a single acidic monomer, sufficient adherability cannot be attained for both of enamel and dentin.

International Publication No. WO 02/092021A1 discloses a dental resin cement composition consisting of a liquid and a powder. A powder-liquid type resin cement, is inferior in handling properties upon mixing as compared with a paste-and-paste type resin cement.

Japanese Patent Publication No. 2005-65902A discloses a dental adhesive composition comprising, as an essential adhesive component, a carboxylic acid compound having one (meth)acryloyl group and one carboxyl group which are bound to an aromatic group as a polymerizable monomer containing a particular carboxylic acid group. However, such the polymerizable monomer having an ester group quickly degrades in an acidic medium.

Dental materials based on polyfunctional amides are known from US 6,953,832 which contain specific polymerizable amides and optionally strongly acidic polymerizable monomers such as dipentaerythritol pentamethacryloyloxy dihydrogenphosphate. Filler containing compositions are suggested. However, US 6,953,832 does not disclose a composite.

### Summary of the Invention

It is an object of the present invention to provide dental compositions, in particular dental cements, which are useful as filling materials, cavity liners and bases, cements, pit and fissure sealants to prevent caries, as adhesive between tooth structure and/or bone and polymeric composites, whereby the dental composition has excellent storage stability and long term mechanical resistance, and whereby the composition may be applied directly on the dental surface.

A further object is to provide dental restorative/prosthetic compositions that are relatively inexpensive and easy to manufacture.

The present invention provides a dental composition comprising
(i) a water-soluble polymerizable compound of the following formula (1),

   AXₙ (1)

   wherein
   - A: is a straight chain or branched linker group containing at least n nitrogen atoms, whereby the linker group has polyamide, polyoxyalkylene and/or polyalkylene imine repeating units and optionally one or more acidic groups,
   - X: are moieties containing a polymerizable double bond, which X may be the same or different and are represented by the following formula (2), whereby a moiety X of formula (2) is linked to the nitrogen atom of an amino group or a carboxamide group of A, 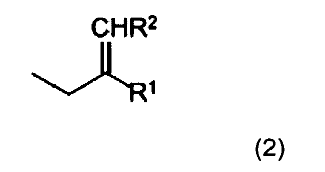
   wherein
   - R¹ and R²: are independent from each other and represent a hydrogen atom, a C₁₋₆ alkyl group or a group -(CH₂)ₘ-COOM, wherein M is a hydrogen atom or a metal atom, and m is an integer of from 0 to 6,
   - L: is a bond or a C₁₋₆ alkylene group; and
   - n: is an integer of at least 1;
   provided that at least one X cannot be a (meth)acryl group; and
(ii) an initiator system;
(iii) optionally a polyacidic polymer; and
(iv) optionally water and/or a water soluble solvent,
(v) optionally a particulate filler.

Moreover, the present invention provides a use of the compound of formula (1) as defined above, in particular for the preparation of a dental composition.

Moreover, the present invention provides a process for preparing a compound of formula (1), which comprises
(i) a step of a step-growth polymerization of a mixture containing a polyamine and a compound having at least two carboxylic acid groups or an anhydride thereof, optionally in the presence of a compound of the following formula (5) for obtaining a polyamide: 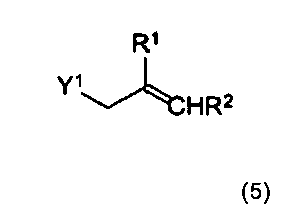 wherein L, R¹ and R² are as defined in claim 1, and Y¹ is a leaving group or Y¹ forms an hydrolysable intramolecular group together with a carboxyl group present in R¹ or R² and the adjacent carbonyl group; and
(ii) a step of introducing moieties of the formula (2) by reacting the polyamide obtained in step (i) with a compound of formula (5) wherein Y¹ is a leaving group and R¹ and R² are as defined in claim 1; or
(iii) a step of reacting a mixture containing a polyamine and a compound of formula (5) for obtaining an amine reaction product; and
(iv) a step of a step-growth polymerization of a mixture containing the amine reaction product obtained in (iii) and a compound having at least two carboxylic acid groups or an anhydride thereof for obtaining the water-soluble polymerizable compound of the formula (1).

The dental compositions according to the invention contain a mixture of hydrolysis-stable polymerizable components including a compound o formula (1). Preferably, the mixture contains at least a crosslinking polymerizable monomer and an acidic polymerizable monomer. The polymerizable monomers are hydrolysis-stable. Specifically, the polymerizable monomers do not contain groups such as ester groups, in the main chain which hydrolyze in aqueous media at pH 3 at room temperature within one month.

### Description of the preferred embodiments

The dental composition of the present invention comprises a polymerizable compound of the following formula (1).

AXₙ (1)

The polymerizable compounds of formula (1) comprise a moiety A, and at least one substituent X. In formula (1), A is a straight chain or branched linker group containing least n nitrogen atoms, whereby the linker group haspolyamide, polyoxyalkylene and/or polyalkylene imine repeating units and optionally one or more acidic groups. The linker has a valency of at least one which corresponds to the total number of substituents X. Accordingly, A may be preferably monovalent (n=1), divalent (n=2), trivalent (n=3), tetravalent (n=4), pentavalent (n=5), or hexavalent (n=6). Preferable A is divalent or trivalent, most preferably divalent.

Preferably, the linker group may be a linear or branched monomeric, oligomeric, polymeric or copolymeric group containing nitrogen atoms at the terminal positions for forming an amide or imine bond with a moiety X. A monomeric groups is a low-molecular group having a molecular weight of up to 500. An oligomeric group is a group having a molecular weight of more than 500 to up to 10000 and may be prepared by a polymerization reaction. A polymeric or copolymeric group is a group having a molecular weight of more than 10000 which may be obtained by a polymerization reaction. The polymerization may be a condensation or addition polymerization.

According to a further preferred embodiment, the polymerizable compound of formula (1) contains one or more acidic groups selected from carboxylic acid groups, phosphonic acid groups, sulfonic acid groups or phosphoric acid ester groups.

In formula (1), X are moieties containing a polymerizable double bond and forming an amide or imine bond with a nitrogen atom of A, which X may be the same or different and are represented by the following formula (2) whereby a moiety X of formula (2) is linked to the nitrogen atom of an amino group or a carboxamide group of A

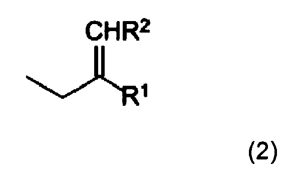

In formula (2), R¹ and R² are independent from each other and represent a hydrogen atom, a C₁₋₆ alkyl group or a group -(CH₂)ₘ-COOM, wherein M is a hydrogen atom or a metal atom and m is an integer of from 0 to 6. The metal atom may be an alkali metal atom or an alkaline earth metal. An alkali metal atom may be sodium or potassium. An alkaline earth metal may be calcium or magnesium. In case of an alkaline earth metal, the second charge on the metal atom is neutralized by either a further carboxylic acid anion or another anion. Preferably, R¹ is a hydrogen atom or a methyl group. Preferably, R² is a hydrogen atom or a group -(CH₂)ₘ-COOH, wherein m is 0, 1 or 2.

In formula (2), L is a bond or a C₁₋₆ alkylene group, preferably a single bond or a methylene or ethylene group.

The linker group imparts water solubility to the compound of formula (1). Water solubility within the sense of the present invention means that the compound of formula (1) can be dissolved as a 0.1 percent by weight solution in water at 25 °C. Preferably, the compound of formula (1) of the present invention has a water solubility of at least 2.0 weight % in water at 25 °C.

According to a preferred embodiment, the linker group has polyamide, polyoxyalkylene and/or polyalkylene imine repeating units and optionally one or more acidic groups, wherein the linker group is a linear or branched group represented by the following formula (3), wherein the nitrogen atom of at least two of the termini forms an amide bond with an X moiety:

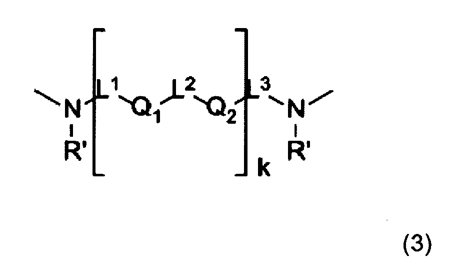

In formula (3), R' represents a hydrogen atom or a substituted or unsubstituted aliphatic or cycloaliphatic hydrocarbon group. Substituents of the aliphatic or cycloaliphatic hydrocarbon group may be selected from hydroxyl groups, thiol groups, amino groups, or carboxylic acid groups or a salt thereof. The R may be the same or different. According to a preferred embodiment, R' is a hydrogen atom. According to a further preferred embodiment, R' is a lower alkyl group having 1 to 6 carbon atoms, more preferably, 1 to 3 carbon atoms.

In formula (3), L¹, L², and L³ may be the same or different. In case a plurality of L¹ an L² are present when k is at least 2, each of L¹ and L² may be the same or different. Preferably, each of L¹ and each of the plurality L² are the same.

L¹, L², and L³ independently represent a single bond, or a C₂₋₂₀ straight-chain, branched or cyclic hydrocarbon group containing from 1 to 6 heteroatoms selected from nitrogen and oxygen in the backbone of the hydrocarbon group, and optionally from 1 to 6 functional groups selected from carboxylic acid groups or a salt thereof, hydroxyl groups, thiol groups and amino groups. In a particular embodiment, L¹, L², and L³ do not carry an optional functional group. Preferably, at least one, more preferably at least two of L¹, L², and L³, do not represent a single bond. Preferably, L¹, L², and L³ contain 1 or 2 heteroatoms selected from nitrogen, and oxygen in the backbone of the hydrocarbon group. Preferably, the hydrocarbon group has 1 to 6 carbon atoms and contains 1 or 2 heteroatoms selected from nitrogen, and oxygen in the backbone of the hydrocarbon group, and optionally from 1 to 3 carboxylic acid groups or a salt thereof.

In formula (3), Q₁ and Q₂, may be the same or different. Q₁ and Q₂ may represent a single bond or a linkage selected from an amide, a urethane, a urea and a thiourea linkage. Preferably, at least one of Q₁ and Q₂ is not a single bond. In case Q₁ and Q₂ represent an amide or urethane linkage, the orientation of the amide or urethane linkage may be the same or different.

In formula (3), k is an integer of at least 0. When k is 0, then L³ cannot be a single bond. Preferably, k is in the range of from 0 to 500, more preferably from 1 to 40.

Preferably, the linker group is a polyamide group obtainable by a process comprising the step of a step-growth polymerization including a condensation reaction or addition reaction of a mixture containing a polyamine having a moiety of the formula (3) and additional hydrogen atoms, and a compound of the following formula (4) having at least two carboxylic acid groups, said carboxylic acid groups may be present in the form of an anhydride:

MOOC-R⁵-COOM (4)

optionally in the presence of a compound of the following formula (5): 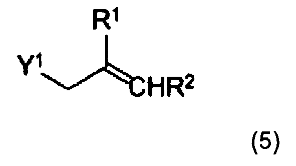 wherein R⁵, R¹ and R² are as defined in above, M is a hydrogen atom or a metal atom which is preferably monovalent, and Y¹ is a leaving group or Y¹ forms an intramolecular anhydride group together with a carboxyl group present in R¹ or R² and the adjacent carbonyl group. The monovalent metal atom is preferably an alkali metal.

The reaction conditions are not particularly limited. According to a preferred embodiment, a polyamine having a moiety of the formula (3) and additional hydrogen atoms is dissolved in a suitable aprotic solvent such as dimethylsulfoxide. Moreover, a compound of formula (4) is separately dissolved in a suitable aprotic solvent such as dimethylsulfoxide. Both solutions are then simultaneously added dropwise at ambient temperature into a round bottom flask. It is preferable to add a suitable stabilizer such as BHT. The reaction mixture is stirred. The reaction time may be from 1 hour to 30 hours. The reaction temperature is preferably in the range of from -10°C to the boiling point of the solvent. Preferably, the reaction is carried out at ambient temperature. The product may be precipitated twice from from a suitable solvent wherein the product is insoluble. As an examples of such a solvent diethylether may be mentioned.

The process for the preparation of the polymerizable compound of the formula (1) according to the present invention comprises (i) a step of a step-growth polymerization of a mixture containing a polyamine and a compound having at least two carboxylic acid groups or an anhydride thereof, optionally in the presence of a compound of the following formula (5) for obtaining a polyamide: 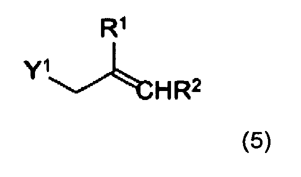 wherein R¹ and R² are as defined above, and Y¹ is a leaving group or Y¹ forms an intramolecular anhydride group together with a carboxyl group present in R¹ or R² and the adjacent carbonyl group.

The process further may further comprise a step of introducing the moieties of the formula (2) by reacting the polyamide obtained in step (i) with a compound of formula (5) wherein Y¹ is a leaving group and R¹ and R² are as defined above; or a step of reacting a mixture containing a polyamine and a compound of formula (5) for obtaining an amide.

The process may also comprise a step of a step-growth polymerization of a mixture containing the amide obtained in (iii) and a compound having at least two carboxylic acid groups or an anhydride thereof for obtaining the water-soluble polymerizable compound of the formula (1).

Preferably, the water soluble polymerizable compound of formula (1) has an average molecular weight of from 300 to 100,000, more preferably 400 to 10,000 Da.

The dental composition according to the present invention comprises an initiator system. The initiator system may be based on a redox initiator or on a photoinitiator.

In case the dental composition contains a redox initiator, the amount of reducing agent and oxidizing agent should be sufficient to provide the desired degree of polymerization. Preferably, the mixed but unset cements of the invention contain a combined weight of about 0.01 to about 10%, more preferably about 0.2 to about 5%, and most preferably about 0.3 to about 3% of the reducing agent and oxidizing agent, based on the total weight (including water) of the mixed but unset cement components. The reducing agent or the oxidizing agent can be microencapsulated as described in U.S. Pat. No. 5,154,762. This will generally enhance shelf stability of the cement parts and if necessary permit packaging both the reducing agent and oxidizing agent together. Water-soluble and water-insoluble encapsulants can be employed. Suitable encapsulating materials include cellulosic materials as cellulose acetate, cellulose acetate butyrate, ethyl cellulose, hydroxymethyl cellulose and hydroxyethyl cellulose being preferred. Other encapsulants include polystyrene, copolymers of polystyrene with other vinylic monomers and polymethylmethacrylate, copolymers of methylmethacrylate with other ethylenically-unsaturated monomers. Preferred encapsulants are ethylcellulose and cellulose acetate butyrate. By varying the choice of encapsulant and the encapsulation conditions, the onset of curing can be tailored to start at times ranging from seconds to minutes. The ratio of amount of encapsulant to activator generally ranges from 0.5 to about 10 and preferably from about 2 to about 6.

Suitable oxidizing agents (initiators) include peroxides such as benzoyl peroxide, cumene hydroperoxide (CHP) and tert-butyl hydroperoxide, ferric chloride, hydroxylamine (depending upon the choice of reducing agent), perboric acid and its salts, and salts of a permanganate or persulfate anion. Preferred oxidizing agents are peroxides, potassium persulfate, ammonium persulfate and hydrogen peroxide.

Suitable reducing agents (activators) include ascorbic acid, benzyl thiourea, ferrous chloride, ferrous sulfate, hydrazine, hydroxylamine (depending upon the choice of oxidizing agent) oxalic acid, thiourea, and salts of a dithionite or sulfite anion. Preferred reducing agents include ascorbic acid and ferrous sulfate.

A photoinitiator should be capable of promoting polymerization of the polymerizable groups on exposure to light of a suitable wavelength and intensity. The photoinitiator preferably is sufficiently shelf-stable and free of undesirable coloration to permit its storage and use under typical dental conditions. Visible light photoinitiators are preferred. Suitable visible light-induced and ultraviolet light-induced initiators include an alpha-diketone (e.g., camphorquinone) with or without additional hydrogen donors (such as sodium benzene sulfinate, amines and amine alcohols). The photoinitiator may be present in an amount sufficient to provide the desired rate of photopolymerization. This amount will be dependent in part on the light source, the thickness of the cement layer to be exposed to radiant energy and the extinction coefficient of the photoinitiator. Preferably, mixed but unset photocurable cements of the invention will contain about 0.01 to about 5%, more preferably from about 0.1 to about 2% photoinitiator, based on the total weight (including water) of the mixed but unset cement components.

The dental composition according to the present invention may contain further polymeric components, such as a polymer having acidic groups.

To effect cross-linking or additional cross-linking of the dental composition, one or more comonomers may be included in the dental composition. Suitable comonomers contain at least one polymerizable functional group. Suitable polymerizable functional groups are ethylenically unsaturated groups (e. g. alkenyl groups and preferably vinyl groups). Ethylenically unsaturated groups are polymerisable by a free radical mechanism. Preferred examples are substituted and unsubstituted acrylates, methacrylates, or alkenes.

A dental composition is prepared by mixing the components of the dental composition of the present invention. The components of the dental composition can be combined (such as by mixing or blending) in a variety of manners and amounts in order to form the dental composition of the present invention.

For example, in a dental cement, a concentrated solution of the polymerizable compound, and the initiator system may be mixed with the particulate filler and optionally further components such as an ionomer, at the time of use.

Alternatively, the polymerizable compound, the initiator system, the particulate filler and optionally an ionomer are provided as a freeze-dried or lyophilized powdered blend under conditions in which there is not sufficient water to allow the setting reaction to proceed. Such systems can then be combined with water at the time of use in order to begin the setting reaction. Once the setting reaction has begun, the resultant mixture may be formed into its desired shape, followed by curing and allowing the mixture to fully harden.

The amount of the water-soluble polymerizable compound of formula (1) in a dental composition may be in the range of from 1 to 70 % by weight based on the total weight of the composition. Preferably, the amount is in the range of from 3 to 50 % by weight based on the total weight of the composition.

The reaction mixture may also include a modifying agent such as tartaric acid, for adjusting the working time and a setting time, respectively, when preparing the cement as described in US-A 4,089, 830, US-A 4, 209,434, US-A 4,317, 681 and US-A 4,374, 936. In general, an increase in working time results in an increase in setting time as well.

The "working time" is the time between the beginning of the setting reaction when the ionomer and modified particulate reactive filler are combined in the presence of water, and the time the setting reaction proceeds to the point when it is no longer practical to perform further physical work upon the system, e.g. spatulate it or reshape it, for its intended dental or medical application.

The "setting time" is the time measured from the beginning of the setting reaction in a restoration to the time sufficient hardening has occurred to allow subsequent clinical or surgical procedures to be performed on the surface of the restoration.

In a setting reaction, due to the presence of polymerizable double bonds, a polymerization reaction takes place.

A dental composition according to the present invention may further contain a polyacidic polymer, for example as a ionomer component in a dental cement. The polyacidic polymer may have polymerizable double bonds so that the polyacidic polymer may be crosslinked with the water-soluble polymerizable compound of formula (1).

Polymerizable acids used for preparing polymers useful for glass-ionomer cement systems include alkenoic acids and unsaturated mono-, di-and tricarboxylic acids. Representative alkenoic acids are described, for example, in US-A 4,016, 124, US-A 4,089, 830, US-A 3,655, 605; US-A 4,143, 018; US-A 4,342, 677, US-A 4,360,605, US-A 4,376, 835 and US-A 5,130, 347. Specific examples are acrylic acid, methacrylic acid, maleic acid, fumaric acid, itaconic acid, crotonic acid, and derivatives thereof, such as the acid chlorides thereof, the acid anhydrides thereof and chloro or bromo derivatives thereof. Particularly preferred monomers are acrylic acid, itaconic acid and maleic acid, and the chlorides or anhydrides thereof. The pendent carboxylic acid groups of the ionomer must be sufficient in number or percent by weight to bring about the setting or curing reaction in the presence of the modified particulate reactive and/or non-reactive filler.

Polymerizable double bonds as a source of additional covalent crosslinking, which imparts additional strength to the ultimate dental composition, may be introduced by reacting a portion of the carboxylic acid groups with a bi-functional monomer. Examples of suitable bi-functional monomers include bisacrylamides such as N,N'-diethyl-1,3-bisacrylamido-propan (BADEP), 1,3-bisacrylamido-propan (BAP), and 1,3-bisacrylamido-2-ethyl-propan (BAPEN); acryloyl chloride, methacryloyl chloride, vinyl azalactone, allyl isocyanate, 2-hydroxyethylmethacrylate (HEMA), 2-aminoethylmethacrylate, 2-isocyanatoethyl methacrylate (IEM), acrylic acid, methacrylic acid and N-vinylpyrrolidone. Other examples of suitable bi-functional monomers are described in US-A 4,035, 321 US-A 5,130, 347.

In general, the weight-to-weight ratio of an ionomer to water in a dental cement is from about 1: 10 to about 10: 1. In general, the concentration of ionomer in water ranges from 25 to 75% by weight, and preferably from 40 to 65 percent. The resultant aqueous solution has a weight ratio of polymer to liquid (polymer : liquid) generally ranging from about 1.5 to 8.

The dental composition may optionally comprise water and/or a water soluble solvent. Suitable solvents are nonreactive diluents such as alcohols. As examples of suitable alcohols, ethanol and propanol may be mentioned.

The amount of water and/or a water soluble solvent in the dental composition of the present invention is preferably in the range of from 5 to 90 % by weight, more preferably 10 to 70 percent by weight based on the total weight of the composition.

The dental composition may optionally contain a particulate filler. In a specific embodiment, the particulate filler is reactive with a polyacid in a cement reaction. A "particulate filler" is a powdered metal oxide or hydroxide, mineral silicate, or ion leachable glass or ceramic.

Examples of reactive particulate filler materials include materials commonly known in the art of dental compositions such as calcium or strontium-containing and aluminum-containing materials. Preferably, particulate reactive fillers contain leachable fluoride ions. Specific examples of particulate reactive fillers are selected from calcium alumino silicate glass, calcium alumino fluorosilicate glass, calcium aluminumfluoroborosilicate glass, strontium aluminosilicate glass, strontium aluminofluorosilicate glass, strontium aluminofluoroborosilicate glass. Suitable particulate reactive fillers further include metal oxides such as zinc oxide and magnesium oxide, and ion-leachable glasses, e.g., as described in US-A 3,655,605, US-A 3,814,717, US-A 4,143,018, US-A 4,209,434, US-A 4,360,605 and US-A 4,376,835.

Suitable non-reactive fillers may be selected from fillers currently used in dental restorative compositions.

The filler may have a unimodal or polymodal (e.g., bimodal) particle size distribution. The filler can be an inorganic material. It can also be a crosslinked organic material that is insoluble in the polymerizable resin, and is optionally filled with inorganic filler. The filler can be radiopaque, radiolucent or non-radiopaque. Examples of suitable non-reactive inorganic fillers are naturally-occurring or synthetic materials such as quartz, nitrides such as silicon nitride, glasses derived from, for example Ce, Sb, Sn, Zr, Sr, Ba and Al, colloidal silica, feldspar, borosilicate glass, kaolin, talc, titania, and zinc glass, and submicron silica particles such as pyrogenic silicas. Examples of suitable non-reactive organic filler particles include filled or unfilled pulverized polycarbonates or polyepoxides. Preferably the surface of the filler particles is treated with a coupling agent in order to enhance the bond between the filler and the matrix. The use of suitable coupling agents include gamma-methacryloxypropyltrimethoxysilane, gamma-mercaptopropyltriethoxysilane, gamma-aminopropyltrimethoxysilane, and the like.

The particulate filler usually has an average particle size of from 0.005 to 100 µm, preferably of from 0.01 to 40 µm as measured using, for example, by electron microscopy or by using a conventional laser diffraction particle sizing method as embodied by a MALVERN Mastersizer S or MALVERN Mastersizer 2000 apparatus. The particulate filler may be a multimodal particulate reactive filler representing a mixture of two or more particulate fractions having different average particle sizes. The particulate reactive filler may also be a mixture of particles of different chemical composition. In particular, it is possible to use a mixture of a particulate reactive material and a particulate non-reactive material. The particulate reactive filler may be surface modified by a surface modifying agent.

The dental compositions of the present invention may further contain pigments, free radical scavengers, polymerization inhibitors, reactive diluents, surfactants (such as to enhance solubility of an inhibitor e. g. polyoxyethylene), coupling agents to enhance reactivity of fillers e.g.,3- (trimethoxysilyl) propyl methacrylate, and rheology modifiers.

An example of a suitable free radical scavenger is 4-methoxyphenol. An example of a suitable inhibitor is hydroxytoluene or butylated hydroxytoluene (BHT). The amount of inhibitor may be selected from 0.001 to 2% and preferably from 0.02 to 0.5% based on the total weight of the copolymer/comonomer/water mixture.

Suitable reactive diluents are alpha,beta unsaturated monomers for providing altered properties such as toughness, adhesion, and set time. Suitable alpha,beta-unsaturated monomers may be acrylates and methacrylates such as methyl acrylate, methyl methacrylate, ethyl acrylate, ethyl methacrylate, propyl acrylate, propyl methacrylate, isopropyl acrylate, isopropyl methacrylate, 2-hydroxyethyl acrylate, 2-hydroxyethyl methacrylate (HEMA), hydroxypropyl acrylate, hydroxypropyl methacrylate, tetrahydrofurfuryl acrylate, tetrahydrofurfuryl methacrylate, glycidyl acrylate, glycidyl methacrylate, the diglycidyl methacrylate of bis-phenol A ("bis-GMA"), glycerol mono- and di- acrylate, glycerol mono- and di- methacrylate, ethyleneglycol diacrylate, ethyleneglycol dimethacrylate, polyethyleneglycol diacrylate (where the number of repeating ethylene oxide units vary from 2 to 30), polyethyleneglycol dimethacrylate (where the number of repeating ethylene oxide units vary from 2 to 30 especially triethylene glycol dimethacrylate ("TEGDMA"), neopentyl glycol diacrylate, neopentylglycol dimethacrylate, trimethylolpropane triacrylate, trimethylol propane trimethacrylate, mono-, di-, tri-, and tetraacrylates and methacrylates of pentaerythritol and dipentaerythritol, 1,3-butanediol diacrylate, 1,3-butanediol dimethacrylate, 1,4-butanedioldiacrylate, 1,4-butanediol dimethacrylate, 1,6-hexane diol diacrylate, 1,6-hexanediol dimethacrylate, di-2-methacryloyloxethyl hexamethylene dicarbamate, di-2-methacryloyloxyethyl trimethylhexanethylene dicarbamate, di-2-methacryloyl oxyethyl dimethylbenzene dicarbamate, methylene-bis-2-methacryloxyethyl-4-cyclohexyl carbamate, di-2-methacryloxyethyl-dimethylcyclohexane dicarbamate, methylene-bis-2-methacryloxyethyl-4-cyclohexyl carbamate, di-1-methyl-2-methacryloxyethyl-trimethyl-hexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-metha-cryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-1-chloromethyl-2-methacryloxyethyl4-cyclohexyl carbamate, 2,2'-bis(4-methacryloxyphenyl)propane, 2,2'bis(4-acryloxyphenyl)propane, 2,2'-bis[4(2-hydroxy-3-methacryloxy-phenyl)]propane, 2,2'-bis[4(2-hydroxy-3-acryloxy-phenyl)propane, 2,2'-bis(4-methacryloxyethoxyphenyl)propane, 2,2'-bis(4-acryloxyethoxyphenyl)propane, 2,2'-bis(4-methacryloxypropoxyphenyl)propane, 2,2'-bis(4-acryloxypropoxyphenyl)propane, 2,2'-bis(4-methacryloxydiethoxyphenyl)propane, 2,2'-bis(4-acryloxydiethoxyphenyl)propane, 2,2'-bis[3(4-phenoxy)-2-hydroxypropane-1-methacrylate]propane,and 2,2'-bis[3(4-phenoxy)-2-hydroxypropane-1-acryalte]propane, may be mentioned. Other suitable examples of polymerizable components are isopropenyl oxazoline, vinyl azalactone, vinyl pyrrolidone, styrene, divinylbenzene, urethane acrylates or methacrylates, epoxy acrylates or methacrylates and polyol acrylates or methacrylates. Mixtures of alpha,beta-unsaturated monomers can be added if desired. Preferably, the mixed but unset dental compositions of the invention will contain a combined weight of about 0.5 to about 40%, more preferably about 1 to about 30%, and most preferably about 5 to 20% water, solvents, diluents and alpha,beta-unsaturated monomers, based on the total weight (including such water, solvents, diluents and alpha,beta-unsaturated monomers) of the mixed but unset dental composition components.

Depending upon the application of the dental composition and the manner in which polymerization is achieved, various components of the cement may be packaged differently. For example, in the case of a redox-based system, ingredients of the dental composition composition are divided into two separate packages-the first package containing the copolymer, comonomer, the initiator and water, and the second package containing the reactive filler and the activator. In another embodiment, the first package contains all solid materials (e.g., copolymer, comonomer, reactive filler and if desired, the reducing agent, and the second package contains water and if desired, the initiator. In the case of photoinitiation, the photo-initiator can be included in either the solid (e. g. paste) or liquid parts of the dental composition.

Preferably, the dental composition of the present invention is packaged as a one-pack composition wherein all components are combined in a single composition.

A compound of formula (1) according to the present invention may be used for the preparation of a dental composition. Specifiically, the dental composition may comprise the polymerizable compound of the formula (1),an initiator system, and optionally a particulate filler. The dental composition may be a dental adhesive compositon or a dental restorative composition.

The invention will now be further illustrated by the following Examples.

### Examples

### Example 1

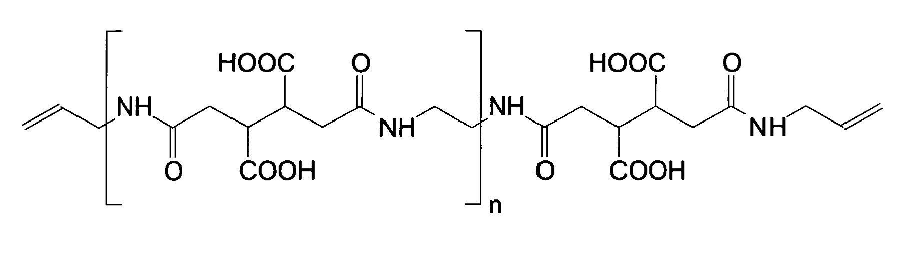

0.005 mol (0.301 g) of ethylene diamine (CAS: 107-15-3) and 0.011 mol (0.628 g) allyl amine (CAS: 107-11-9) are dissolved in 4 mL dimethyl sulfoxide (DMSO). 0,011 mol (2.179 g) of butane tetracarboxylic acid dianhydride are dissolved in 20 mL DMSO. Both solutions are simultanously slowly added dropwise into a glass flask at room temperature over the same amount of time. BHT is added to the mixture as a stabiliser. The reaction mixture is stirred for 16 h at room temperature. The product is precipitated in 300 mL ethyl acetate (CAS: 141-78-6) (13-fold amount based on the amount of DMSO). The precipitate is isolated and dried *in vacuo.*

Calculated degree of polymerization (end group analysis NMR): x≈3.

IR: ν (in cm⁻¹) = 3304, 3077, 2925, 1709, 1627, 1542

¹H-NMR (DMSO-d₆, 400 MHz) = 8.122-7.787 (m, CONH), 5.792-5.705 (m, CH₂C*H*CH₂NH, vinylic protons), 5.186-5.006 (m, CH₂CHCH₂NH, vinylic protons), 3.655-2. 718(m), 2.509-2.080 (m).

### Example 2

Variation of the concentration of allyl amine und ethylene diamine:
The reaction was carried in the same way as in Example 1, except that 0.011 mol (0.661 g) ethylene diamine and 0.0028 mol allyl amine (0.160 g) were used:
   IR: ν (in cm⁻¹) = 3304, 3076, 2922, 1703, 1648, 1542
   ¹H-NMR (DMSO-d₆, 400 MHz) = 12.336 (s, COOH), 8.126-7.792 (m, CONH), 5.801-5.719 (m, CH₂C*H*CH₂NH, vinylic protons), 5.195-5.013 (m, C*H*₂CHCH₂NH, vinylic protons), 3.662-2.718 (m), 2.504-2.090 (m).

## Claims

1. Dental composition comprising
(i) a water-soluble polymerizable compound of the following formula (1),
AXₙ (1)
wherein
A is a straight chain or branched linker group containing at least n nitrogen atoms, whereby the linker group has polyamide, polyoxyalkylene and/or polyalkylene imine repeating units and optionally one or more acidic groups,
X are moieties containing a polymerizable double bond, which X may be the same or different and are represented by the following formula (2), whereby a moiety X of formula (2) is linked to the nitrogen atom of an amino group or a carboxamide group of A, 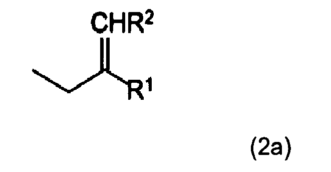
wherein
R¹ and R² are independent from each other and represent a hydrogen atom, a C₁₋₆ alkyl group or a group -(CH₂)ₘ-COOM, wherein M is a hydrogen atom or a metal atom, and m is an integer of from 0 to 6,
L is a bond or a C₁₋₆ alkylene group; and
n is an integer of at least 1;
provided that at least one X cannot be a (meth)acryl group; and
(ii) an initiator system;
(vi) optionally a polyacidic polymer; and
(vii) optionally water and/or a water soluble solvent,
(viii) optionally a particulate filler.

2. The dental cement composition according to claim 1, whereby the linker group having polyoxyalkylene and/or polyalkylene imine repeating units and optionally one or more acidic groups is a linear or branched group represented by the following formula (3), wherein the nitrogen atom of at least two of the termini forms an amide bond with an X moiety; 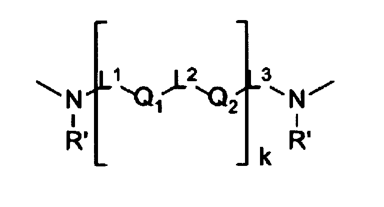 wherein
R' represents a hydrogen atom or a substituted or unsubstituted aliphatic or cycloaliphatic hydrocarbon group, wherein each R may be the same or different;
L¹, L², and L³ which may be the same or different, independently represent a single bond, or a C₂₋₂₀ straight-chain, branched or cyclic hydrocarbon group optionally containing from 1 to 6 heteroatoms selected from nitrogen and oxygen in the backbone of the hydrocarbon group, and optionally from 1 to 6 functional groups selected from carboxylic acid groups or a salt thereof, hydroxyl groups, thiol groups and amino groups, and in case a plurality of L¹ and L² are present, each of L¹ and L² may be the same or different;
Q₁ and Q₂, which may be the same or different, independently represent a single bond or a linkage selected from an amide, a urethane, a urea and a thiourea linkage;
k is an integer of at least 0.

3. The dental composition according to claim 2, wherein Q₁ and Q₂ represent an amide linkage and k is at least 1.

4. The dental composition according to claim 3, wherein L³ represents a polyoxyalkylene group, and k is 0.

5. The dental composition according to any one of claims 1 to 4, wherein the water soluble polymerizable compound of formula (1) has an average molecular weight of from 300 to 10,000.

6. The dental composition according to any one of claims 2 wherein Q₁ or Q₂ is a single bond and the other of Q₁ and Q₂ is selected from an amide, a urethane, a urea and a thiourea linkage, and k is at least 1.

7. A process for the preparation of a water-soluble polymerizable compound of the formula (1) as defined by claim 1 or 2, which comprises
(i) a step of a step-growth polymerization of a mixture containing a polyamine and a compound having at least two carboxylic acid groups or an anhydride thereof, optionally in the presence of a compound of the following formula (5) for obtaining a polyamide: 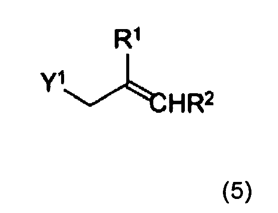 wherein L, R¹ and R² are as defined in claim 1, and Y¹ is a leaving group or Y¹ forms an hydrolysable intramolecular group together with a carboxyl group present in R¹ or R² and the adjacent carbonyl group; and
(ii) a step of introducing moieties of the formula (2) by reacting the polyamide obtained in step (i) with a compound of formula (5) wherein Y¹ is a leaving group and R¹ and R² are as defined in claim 1; or
(iii) a step of reacting a mixture containing a polyamine and a compound of formula (5) for obtaining an amine reaction product ; and
(iv) a step of a step-growth polymerization of a mixture containing the amine reaction product obtained in (iii) and a compound having at least two carboxylic acid groups or an anhydride thereof for obtaining the water-soluble polymerizable compound of the formula (1).

8. The process according to claim 7, wherein the compound having at least two carboxylic acid groups is a compound of formula (4),
MOOC-R⁵-COOM (4)
wherein R⁵ represents a C₁₋₂₀ straight-chain, branched, cyclic or aromatic hydrocarbon group which may optionally contain from 1 to 6 heteroatoms selected from nitrogen, oxygen, or sulfur atoms in the backbone of the hydrocarbon group, and optionally from 1 to 6 functional groups selected from carboxylic acid groups, hydroxyl groups, thiol groups and amino groups, and the M independently represent a hydrogen atom or a metal atom.

9. The process according to claim 7 or 8, wherein the compound of formula (5) is allyl chloride or allyl bromide. PLEASE CHECK

10. A water-soluble polymerizable compound of the following formula (1a),
AXₙ (1a)
wherein
A is a linker group containing at least n nitrogen atoms and optionally one or more acidic groups, which is represented by the following formula (3), wherein the nitrogen atom of at least two of the amine termini forms an amide bond with an X moiety; 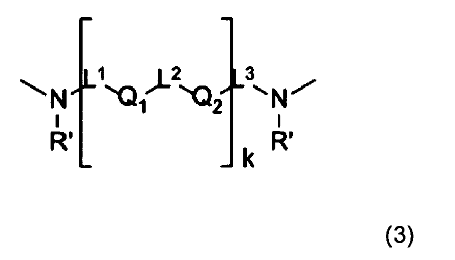 wherein
R' represents a hydrogen atom or a substituted or unsubstituted aliphatic or cycloaliphatic hydrocarbon group, wherein each R' may be the same or different;
L¹, L², and L³ which may be the same or different, independently represent a single bond, or a C₂₋₂₀ straight-chain, branched or cyclic hydrocarbon group containing from 1 to 6 heteroatoms selected from nitrogen and oxygen atoms in the backbone of the hydrocarbon group, and optionally from 1 to 6 functional groups selected from carboxylic acid groups or a salt thereof, hydroxyl groups, thiol groups and amino groups, and in case a plurality of L¹and L², are present, each of L¹ and L² may be the same or different;
Q₁ and Q₂, which may be the same or different, independently represent a single bond or a linkage selected from an amide, a urethane, a urea and a thiourea linkage;
k is an integer of at least 0;
X are moieties containing a polymerizable double bond, which X may be the same or different and are represented by the following formula (2), whereby a moiety × of formula (2) is linked to the nitrogen atom of an amino group of A, 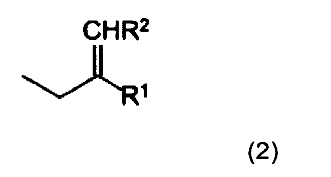
wherein
R¹ and R² are independent from each other and represent a hydrogen atom, a C₁₋₆ alkyl group or a group -(CH₂)ₘ-COOM, wherein M is a hydrogen atom or a metal atom, and m is an integer of from 0 to 6,
L is a bond or a C₁₋₆ alkylene group; and
n is an integer of at least 2;
wherein the linker group is a linear or branched group.

11. Use of a compound of formula (1) as defined in any of claims 1 to 6, for the preparation of a dental composition.
